(19) Europäisches Patentamt
European Patent Office
Office européen des brevets

(11) **EP 4 041 159 B1**

(12) **EUROPEAN PATENT SPECIFICATION**

(45) Date of publication and mention
of the grant of the patent:
**08.01.2025 Bulletin 2025/02**

(21) Application number: **20793074.4**

(22) Date of filing: **08.10.2020**

(51) International Patent Classification (IPC):
*A61F 13/00* (2024.01)        *A61F 13/08* (2006.01)

(52) Cooperative Patent Classification (CPC):
**A61F 13/00051; A61F 13/08**

(86) International application number:
**PCT/GB2020/052494**

(87) International publication number:
**WO 2021/069899 (15.04.2021 Gazette 2021/15)**

(54) **SENSOR APPARATUS FOR A COMPRESSION GARMENT**

SENSORVORRICHTUNG FÜR EIN KOMPRESSIONSKLEIDUNGSSTÜCK

APPAREIL CAPTEUR POUR UN VÊTEMENT DE COMPRESSION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **08.10.2019 GB 201914534**

(43) Date of publication of application:
**17.08.2022 Bulletin 2022/33**

(73) Proprietor: **Oxford Healthtech Ltd.
Berkshire RG14 1QL (GB)**

(72) Inventors:
• **HUQ, Syed Ejazul
Newbury Berkshire RG14 1QL (GB)**
• **AL AIOUBI, Mohamad Yasser
Newbury Berkshire RG14 1QL (GB)**

(74) Representative: **Marks & Clerk LLP
15 Fetter Lane
London EC4A 1BW (GB)**

(56) References cited:
**US-A1- 2017 079 868     US-A1- 2017 100 300
US-A1- 2019 151 160**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

BACKGROUND OF THE INVENTION

The present invention relates to a sensor apparatus for a compression garment.

Compression therapy (CT) via the wearing of an elasticated compression garment (CG) is widely used for the treatment and management of blood circulatory problems. These include venous deficiencies, which can cause venous leg ulcers (VLU) and deep vein thrombosis (DVT). Compression therapy also assists in the movement and removal of trapped fluid in the body's lymphatic system which can help to reduce or prevent lymphoedema. Other medical applications of compression therapy include after liposuction surgery, abdominoplasty, facelift, face compression, neck compression, Veronique compression, for the control of loose skin, after a Brazilian Butt Lift (BBL), after Caesarean section (C-section), hernia surgery or hysterectomy.

Compression garments are also known to provide ergogenic benefits during sports & athletics training and performance. Proposed benefits include an increase in strength and power as well as improved endurance performance. Advantages are thought to be achieved via a number of mechanisms, which include an increase in muscle oxygenation resulting from improved blood flow to the muscle and a reduction in blood lactate levels and muscle oscillation, thought to reduce the severity of fatigue and help in post exercise recovery.

To work optimally, compression garments should provide a predetermined amount of compression (pressure) to the relevant body part of the wearer. For this reason, compression garment, such as compression stockings, typically come in different sizes, to suit differently sized wearers. However, because different body parts have different shapes and sizes, readymade garments do not always provide the required predetermined compression. For this reason, it can be desirable to have apparatus suitable for determining information relating to the actual amount of pressure a compression garment is applying to a wearer.

It is consequently an aim of the present invention to provide a novel approach for sensing one or more properties of a compression garment.

D1 (US 2017/0079868 A1) relates to a compressive pressure device, having an inner compressive pressure sleeve and an overlying outer compressive pressure wrap and a sensor located (a) between the body and the sleeve, (b) within the sleeve, (c) between the sleeve and the wrap, or (d) within the wrap.

D2 (US 2019/0151160 A1) relates to a sensor for measuring a pressure applied by a bandage.

D3 (US 2017/0100300 A1) relates to a compression garment with compression applying straps, with sensors for sensing the compression applied to the straps.

SUMMARY OF THE INVENTION

When viewed from a first aspect, the invention provides a compression garment apparatus comprising a compression garment and a sensor apparatus, wherein the sensor apparatus comprises a sensor device, attached to an outer surface of the compression garment, and a controller, wherein the sensor device comprises a first mounting point, attached to a first point on the outer surface of the compression garment, and a second mounting point, attached to a second point on the outer surface of the compression garment; wherein the sensor device is arranged to sense displacement between the first and second mounting points; and wherein the controller is configured to process information representative of the sensed is placement to estimate a pressure exerted by the compression garment on a wearer of the compression garment.

From a further aspect, the invention provides a sensor apparatus comprising a sensor device, for attaching to an outer surface of a compression garment, and a controller, wherein the sensor device comprises: a first mounting point for attaching to a first point on the outer surface of the compression garment; and a second mounting point for attaching to a second point on the outer surface of the compression garment, wherein the sensor device is arranged to sense displacement between the first and second mounting points; and wherein the controller is configured to process information representative of the sensed displacement to estimate a pressure exerted by the compression garment on a wearer of the compression garment.

Thus it will be appreciated that the present invention provides a means of determining a displacement, or stretch, in a compression garment using a sensor device attached to the *outside* of the compression garment. This displacement (e.g. corresponding to a change in circumference of the garment) can be used to estimate the pressure exerted by the compression garment on the wearer's body, but it does not require anything to be placed *between* the compression garment and the wearer. As the sensor is mounted on the outside of the compression garment, rather than between the compression garment and the wearer's skin, the sensor does not itself cause any additional expansion or distortion of the compression garment, which might otherwise lead to inaccurate pressure indications-e.g. by causing a hump in the garment over the sensor, which could create additional localised pressure or shear forces on the body. It also enables the sensor to be easily attached to and detached from the compression garment, and minimises wearer discomfort.

The controller may be configured to receive the information representative of the sensed displacement from the sensor device-e.g. as an analogue signal or as digital data.

The controller may be attached to the compression garment along with the sensor device. The controller may be integrated with the sensor device as a single device-e.g. both being on a common structure, or in a common housing or casing with the sensor device. Thus the sensor device may comprise the controller. Alternatively, the controller may be separate (e.g. remote) from the sensor device. The controller may be provided as a separate control unit. The controller may be communicatively connected to the sensor device (e.g. by a cable or other wired or wireless communication link).

The compression garment may comprise an inner surface which may be in contact with a body of the person or animal. The outer surface will then face away from the person or animal.

The compression garment may be a woven or fabric compression garment. It may be a compression stocking or other garment. It may be shaped to conform to a human or animal limb or other body part-e.g. a leg or an arm. It may be suitable for use in compression therapy-e.g. for reducing the risk of deep vein thrombosis. It may be an item of sportswear.

Each mounting point may be attached to the compression garment using a mechanical fastener and/or chemical bonding. The sensor device may be configured for attachment to the compression garment using any one or more of: hook-and-loop pads, a popper, a magnet, stitching, adhesive, riveting, welding, or any other suitable mechanism.

In some embodiments, the sensor device comprises a third mounting point attached to a third point on the outer surface of the compression garment. This additional mounting point provides additional stability to avoid twisting of the sensor.

The sensor device may be non-destructively detachable from the compression garment. This means that the garment can easily be washed and reused without damaging the sensor. Furthermore, sensor readings may be taken before and after washing of the garment in order to determine any loss of compression pressure caused by the washing process. It also allows one sensor to be used on multiple compression garments at different times, which may be more economical than disposing of the sensor with the garment.

The garment may be elongate (e.g. broadly cylindrical) and the sensor device may be attached to the garment with the first and second mounting points positioned about a common circumference of the garment. The mounting points may be the same distance from an end of the garment-e.g. from a foot of a compression stocking. They may be a common distance along a limb of a wearer when the garment is being worn. This may be a clinically recommended distance. In this way, the sensor can be used to measure circumferential displacement, or tension, in the garment. The mounting points may be just above the ankle for compression garments (e.g. a compression stocking) used for venous leg ulcer management, or any other appropriate location for other suitable or desired applications, such as lymphoedema or deep vein thrombosis management.

The sensor device may be used to sense a first displacement at a first time, which may be a time when the compression garment is not being worn. It may be used to sense a second displacement at a second time, which may be a time when the compression garment is being worn. In this way, the sensor device may be used to detect when the garment is and/or is not being worn, or may be used to determine whether the compression garment is correctly applied.

The sensor device may have an initial state, which the sensor device is in when it is attached to a slack compression garment (i.e. when the garment is not being worn). In this initial state, the first and second mounting points may be less than 50mm, 30mm, 20mm, 10mm or 1mm apart. A relatively small initial distance can help reduce error -e.g. if there is non-uniform pressure in the garment-as well as facilitating a compact sensor device. The sensor device may be such that the first and second mounting points are less than 70mm, 50mm, 40mm, 20mm or 10mm apart when the garment is under a maximum specified tension for the garment in normal usage-e.g. when worn by a human of an appropriate size for the compression garment,

The sensor device may comprise a first part and a second part, wherein the first part is moveable relative to the second part. The first mounting point may be on the first part, and the second mounting point may be on the second part. The first and second parts may be connected (e.g. by a thread), or they may be unconnected. The first part may comprise a housing which may house one or more electrical components, which may include the controller.

The sensor device may comprise a biasing member (e.g. a spring) arranged to resist displacement of the first mounting point away from the second mounting point in one or more directions. It may be arranged to urge the first mounting point towards the second mounting point, at least when the second mounting point is displaced from the first mounting point by more than a minimum displacement. In some embodiments, the biasing member is arranged to bias the sensor device into or towards the initial state of the sensor device. The biasing member may comprise a first fixing point and a second fixing point. The first fixing point of the biasing member may be fastened to the first part. The second fixing point of the biasing member may be fastened to the second part.

In some embodiments, the biasing member is elongate (e.g. a straight or coiled spring) and a first end and a second end of the biasing member are both fastened to the first part and are separated by a fixed distance, and a third (e.g. central) point or portion of the biasing member, between the first and second ends, is fastened to the second part of the sensor device. The biasing member may be arranged such that a displacement of the first part away from the second part causes a deflection and/or elongation of the biasing member, which may be resisted by the biasing member, so as to resist the displacement.

Such a biasing member can help to ensure that, when the compression garment is removed from the wearer, the first and second mounting points reliably return to the same initial (e.g. slack) state. This can help to reduce disadvantageous effects of hysteresis when the sensor device is reused and repeatedly cycled between an initial state when the garment is not being worn, and a tensioned state when the garment is being worn.

The sensor device may generate an analogue or digital electronic signal representative of displacement between the first and second mounting points. The signal may be representative of absolute displacement and/or representative of a change in displacement (i.e. relative displacement). In some embodiments, the sensor device may generate a voltage that is representative of-e.g. proportional to-the displacement between the first and second mounting points. It will be appreciated that this signal can relate to a pressure exerted by the compression garment-e.g. on a wearer of the garment.

The sensor device may output a signal (e.g. an optical or electronic signal) that is representative of the displacement-e.g. for processing by the controller when the controller is separate from the sensor device. In some embodiments, the controller is integral with or part of the sensor device, in which case the sensor device may send such a signal to the controller over an internal connection or interface.

The controller may comprise a microcontroller or other processing device for executing software instruction, and/or dedicated hardware circuitry (e.g. an ASIC or FPGA), which may be configured for processing a signal representative of the displacement. The controller may be configured to estimate pressures in the range mHg to 100 mmHg. It may log data representative of the displacement and/or pressure over time at intervals, which may be regular intervals.

The information representative of displacement may be represented or encoded as an analogue or digital signal or data, in any appropriate way. The controller may process the information representative of the sensed displacement as digital data. The controller may be configured to process the information representative of displacement to determine a tension force, or a change in a tension force, in the compression garment, e.g. as a digital tension value, which it may store in a memory of the controller. The controller may be configured to process the information representative of displacement to determine an estimate of the displacement, or a change in the displacement, between the first and second mounting points, e.g. as a digital displacement value, which it may store in a memory of the controller. It may determine the digital displacement value by processing the digital tension value.

The controller may be configured to determine an absolute or relative tension force value and/or an absolute or relative displacement value using a look-up table stored in a memory of the controller. It may use the information representative of displacement to access an entry from the look-up table. The data in the look-up table may be empirically determined.

The controller may be configured to determine an absolute or relative tension force value and/or an absolute or relative displacement value by numerically evaluating a function, which may be a function of the information representative of displacement. It may do so by processing one or more algorithms.

The controller may be configured to process an absolute or relative tension force and/or an absolute or relative displacement to estimate a pressure exerted by the compression garment on a wearer of the garment.

The controller may estimate the pressure exerted by the compression garment on a wearer of the garment using a look-up table, or by evaluating an algorithm, wherein the look-up table or algorithm implement the Laplace equation.

In some embodiments, the controller is configured to calculate the pressure exerted by the compression garment by:

determining a tension force in the compression garment;
determining a displacement between the first and second mounting points; and
calculating the pressure as the determined tension divided by a linear function of the determined displacement.

The linear function of the determined displacement may be the sum of a first constant and a product. The product may be the product of a second constant and the determined displacement. The first constant may be equal to the circumference of the compression garment in a slack or un-stretched, e.g. initial, state. In embodiments where a biasing member (e.g. a spring) is provided to assist in bringing the sensor device into an initial (un-stretched) state, determining the tension in the compression garment may comprise subtracting from the tension force a biasing force provided by the biasing member. The biasing force may be determined (e.g. calculated) using the determined absolute or relative displacement between the first and second mounting points. However, in some embodiments, the biasing force provided by the biasing member may be negligible in comparison to the tension in the compression garment, and so may be disregarded in the estimation of the pressure exerted by the garment.

The sensor device or separate controller may be configured to output a signal if the displacement between the first and second mounting points and/or the pressure is greater than a predetermined value.

The sensor device may use any suitable means for sensing displacement. It may sense displacement optically and/or electronically.

In one set of embodiments, the sensor device comprises a light-emitting component (e.g. an LED) and a light-receiving transducer, such as a photo detector (e.g. a photodiode, photoresistor, or phototransistor). The sensor device may be arranged such the amount of light received by the light-receiving transducer from the light-emitting component depends on the displacement between the first and second mounting points-e.g. being proportional thereto.

Light may travel partly or entirely through free space between the light-emitting component and light-receiving transducer. However, in some embodiments, one or more optical fibres may extend at least partly between the light-emitting component and light-receiving transducer. The sensor device may comprise an optical fibre one end of which is in a fixed relationship to one of the light-emitting component and light-receiving transducer, and an opposite end of which is in a moveable relationship to the other of the light-emitting component and light-receiving transducer. The sensor device may be configured so that increased displacement between the first and second mounting points causes less light to strike the light- receiving transducer, resulting in a lower output signal from the transducer.

The sensor device may comprise a first optical fibre and a second optical fibre, which may be arranged in series. The first optical fibre may be arranged to receive light emitted from the light-emitting component and to transmit the light to the second optical fibre. The second optical fibre may be arranged to transmit the light received from the light emitting component (via the first optical fibre) to the light-receiving transducer. The sensor device may be configured so that increased displacement between the first and second mounting points causes less light to be transmitted from the first optical fibre to the second optical fibre, thereby causing less light to strike the light receiving transducer, resulting in a lower output signal from the transducer.

The first optical fibre and the second optical fibre may be coaxial (i.e. extending along the same axis), along at least respective parts or all of their lengths, in an initial (e.g. un-stretched) state of the compression garment. The first optical fibre and the second optical fibre may be moveable relative to each other- e.g. being carried respectively on the aforesaid first and second parts. They may be arranged such that relative movement between the first optical fibre and the second optical fibre causes the optical fibres to misalign, thereby reducing the amount of light transmitted from the light emitting component to the light receiving transducer via the first and second optical fibres. This can result in a lower output signal from the transducer.

The sensor device may be configured so that displacement between the first and second mounting points causes a light-emitting end of the first fibre to move relative to a light-receiving end of the second fibre. The movement may be parallel to an axis of the first and/or second fibres (e.g. increasing a separation between the ends) and/or it may be lateral or perpendicular to an axis of the first and/or second fibres (e.g. reducing an overlap area between the ends).

The second part of the sensor device may comprise an arm that extends from the second part of the sensor device towards the first part of the sensor device. The sensor device may comprise a third part, which may be arranged at the distal end of the arm (i.e. towards the first part of the sensor device). The second and third parts of the sensor device may be connected so that they are in a fixed spatial relationship. The third part may be arranged adjacent the first part. Thus, in such embodiments, displacement of the second part relative to the first part (e.g. caused by stretching of the garment), results in a corresponding displacement of the third part relative to the first part. The first optical fibre may be arranged on the first part and the second optical fibre may be arranged on the third part.

In some embodiments the light-emitting component and light-receiving transducer are moveable relative to each other-e.g. being carried respectively on the aforesaid first and second parts.

The light-emitting component and light-receiving transducer may be elongate components and may be arranged in at least partly overlapping relationship. They may be arranged so that the length of overlap depends on displacement between the first and second mounting points. They may comprise an organic LED and/or an organic photodetector.

In some embodiments the light-emitting component and light-receiving transducer are in a fixed spatial relationship (e.g. being both on the first part), but the light- emitting component is configured to output light to an optical component (e.g. an optical fibre or a mirror) that is moveable relative to the light-emitting component or to the light-receiving transducer (e.g. being coupled to the second part). In this way, it is possible to cause the amount of light that reaches the light-receiving transducer to vary as the distance between the first and second mounting points varies. For example, the second part (e.g. the second mounting point) may be coupled to a point on an optical fibre such that relative movement of the second part causes the optical fibre to bend so that the ends of the optical fibre are drawn closer together (or are pushed further apart). In this way the amount of light transmitted from the light-emitting component to light-receiving transducer will vary.

This arrangement allows the powered components of the sensor device (e.g. the light-emitting component and the light-receiving transducer) to remain a fixed distance from each other, while a non-powered component is arranged to move as the garment is stretched. If the light-emitting component and the light-receiving transducer were attached to the first mounting point and the second mounting point respectively, a flexible power transmission system between the two components would be required in order to accommodate the changing distance between them during stretching of the garment.

In some embodiments the light-emitting component and light-receiving transducer are in a fixed spatial relationship (e.g. being both on the first part), but the sensor device comprises a light-interrupting element, such as a wedge, configured to progressively occlude an optical path between the light-emitting component and light-receiving transducer in dependence on the displacement between the first and second mounting points.

In a second set of embodiments, the sensor device uses a variable capacitance to sense displacement between the first and second mounting points. The sensor device may comprise a first capacitor plate coupled to the first point, and a second capacitor plate coupled of the second point. The plates may be in a common plane. This may help to keep the sensor device thin and unobtrusive. The plates may be thin-film plates (e.g. MEMS-type micro-fabricated capacitor plates). They may comprise a plurality of interleaved protrusions. The sensor device, or a remote controller, may comprise circuitry for measuring the capacitance between the first and second plates, and optionally for calculating a displacement value and/or pressure value therefrom.

In a third set of embodiments, the sensor device uses a variable resistance to sense displacement between the first and second mounting points. The sensor device may comprise a displacement-sensing resistor, such as a strain gauge, conductive polymer, or piezoresistive element. A first end of the resistor may be coupled to the first point, and a second end may be coupled of the second point. The sensor device, or a remote controller, may comprise circuitry for measuring thresistance across the resistor, and optionally for calculating a displacement value and/or pressure value therefrom.

In a fourth set of embodiments, the sensor device uses a variable magnetic field to sense displacement between the first and second mounting points. The sensor device may comprise a magnet and a magnetic field sensor, such as a Hall-effect or Lorentz-effect sensor, which may be a MEMS sensor. The magnet may be coupled to the first point, and the magnetic field sensor may be coupled of the second point. The sensor device, or a remote controller, may comprise circuitry for measuring the magnetic field, and optionally for calculating a displacement value and/or pressure value therefrom.

In a fifth set of embodiments, the sensor device uses a variable electric field to sense displacement between the first and second mounting points. The sensor device may comprise an electric field generator (e.g. a coil) and an electric field sensor (e.g. another coil), which may be a MEMS sensor. The electric field generator may be coupled to the first point, and the electric field sensor may be coupled of the second point. The sensor device, or a remote controller, may comprise circuitry for measuring the electric field, and optionally for calculating a displacement value and/or pressure value therefrom.

The sensor device may comprise a cover or housing, which may protect one or more optical or electrical components from damage and/or from ambient light exposure.

The sensor device may be compact, so as to be unobtrusive to use. It may have a maximum thickness of 50mm, 20mm, 10mm, 1mm or less. It may have a maximum dimension or diameter of 100mm, 50mm, 20mm, 10mm or less.

The sensor device may comprise a power supply, or it may comprise a port (e.g. an electrical connector) for receiving power from an external source.

In some embodiments, the compression garment may comprise a plurality of layers and the sensor device may be attached to an outer surface of one layer of the compression garment, which need not necessarily be the outermost layer. Thus, it is possible that the sensor device may be embedded within the compression garment.

The sensor apparatus and/or compression garment apparatus may comprise a plurality of sensor devices arranged to sense displacement between a plurality of pairs of points on the compression garment-e.g. at the ankle, mid-calf and proximate the knee. The sensor device or a separate controller may be configured to determine a graduated pressure profile, or 2D or 3D map of pressure, from data output by the plurality of sensor devices. This may be useful in a compression garment for venous leg ulcer or management where at least three sensors may be used to determine the pressure at the ankle, mid-calf and just below the knee taking the geometry of the leg into consideration and using appropriate analytical expression where the information generated by the individual sensors may be combined in an electronic device (e.g. the controller) to help generate and display a compression profile. The controller may be configured to determine a rate of blood flow or a change in muscle volume from a plurality of sensor devices; this may be useful for assessment of performance of an athlete.

A sensor apparatus according to the present invention may comprise the controller and one or more further sensor devices, each further sensor device comprising respective first and second mounting points for attaching to respective first and second points on the outer surface of the compression garment. Each further sensor device may be arranged to sense displacement between each of the respective first and second mounting points. The controller may be configured to determine a rate of blood flow or a change in muscle volume from the respective displacements. The sensor devices may be used on a properly functioning and a damaged leg to assess compartment syndrome.

The controller and/or drive electronics and/or a power supply (e.g. a battery or cell) may be an integral part of the sensor device, or may be a separate entity, e.g. worn separately from the sensor.

Information determined by the sensor device may be stored in the sensor device, e.g. in a readable memory chip, or may be transmitted (e.g. wirelessly) to a separate controller or electronic device, such as a mobile phone, PC, etc. where it may be stored and/or processed and/or displayed.

Information relating to a measure of displacement and/or pressure of the garment may be determined and stored, e.g. on the controller. Such information may be stored at intervals, which may be regular intervals.

Data derived from the sensor device may be logged. The data may be logged by the controller or by another device. By logging data representative of the pressure or tension in the compression garment, data relating to when and/or how long the garment has been worn for may be obtained. This may be used by clinicians to obtain an indication of patient compliance and/or an indication of an effectiveness of the compression garment at different pressures. For example, it may be possible to deduce from the obtained pressure measurements that a compression garment is not being worn as often as recommended, or is being worn incorrectly. This can be useful because patients suffering from medical conditions like lymphoedema, venous leg ulcers, DVT etc. may avoid wearing compression garments as prescribed by clinicians because the garments can be restrictive and uncomfortable to wear. Thus, it is often the case that patients do not wear the CGs for the prescribed duration. This not only means that the patient is likely to continue to suffer, but also that the clinician and/or nursing staff may be misled, e.g. by making an unnecessary change in treatment or, in extreme cases, performing surgery on the patient.

While the sensor apparatus of the present invention has been described above as suitable for use on a compression garment, the Applicant has appreciated that the sensor apparatus may be used for other applications.

In one set of embodiments, the object comprises a nappy (diaper). The Applicant has appreciated that, by sensing the displacement of a surface (e.g. outer surface) of a nappy (diaper), an indication of a state of fullness of the nappy (diaper) may be determined. The sensor device may thus be arranged to detect expansion of the nappy due to absorption by the nappy of urine and/or the presence of faecal matter in the nappy. In this set of embodiments, the sensor device may implement any of the features disclosed herein. It may be permanently or detachably fastened to an outer surface of the nappy. It may comprise an integral controller and power supply as disclosed herein.

The sensor device may comprise an output for providing an indication of fullness of the nappy (diaper). The output may comprise a speaker or a light-emitting component, e.g. an LED. The output may comprise electronic circuitry, such as a radio transmitter, configured to transmit a signal, e.g. to a smartphone e.g. of a parent, guardian or carer. The indication may be binary (e.g. full or not full), or may indicate a degree of fullness of the nappy.

The sensor device may be configured to determine when the sensed displacement in the nappy (diaper) exceeds a predetermined threshold. The output of the sensor device may be configured to provide an indication, e.g. transmit a signal, in response to determining that the sensed displacement exceeds the predetermined threshold. In this way, the sensor device may be used to provide an alert to a parent, guardian or carer that a nappy (diaper), e.g. worn by a baby, infant, toddler, child, adult or animal, has been filled, e.g. by urine or faeces, and needs to be replaced.

A plurality of sensor devices may be attached to different locations on the outer surface of the nappy (diaper). This may improve the accuracy of the indication of the fullness of the nappy. Furthermore, the displacements measured by the plurality of sensor devices may be used to determine whether the nappy has been filled by liquids (e.g. urine) or solids (e.g. faeces).

The nappy (diaper) may be disposable or reusable.

However, the object may be any object of which it is suitable or desirable to sense the displacement or stretch.

The sensor device may be used to determine information relating to a pressure exerted by the object on another entity such as a human or animal which is in contact with the object.

Features of any aspect or embodiment described herein may, wherever appropriate, be applied to any other aspect or embodiment described herein. Where reference is made to different embodiments or sets of embodiments, it should be understood that these are not necessarily distinct but may overlap.

BRIEF DESCRIPTION OF THE DRAWINGS

Preferred embodiments of the invention will now be described, by way of example only, with reference to the accompanying drawings, in which:

Figure 1 is a perspective view of a pressure sensor device according to an embodiment of the present invention;
Figures 2a-d are cross-sectional front and plan views of the pressure sensor device of Figure 1;
Figure 3 is a schematic cross section showing the change in circumference of a garment when stretched;
Figures 4a-d are cross-sectional front and plan views of a pressure sensor device according to a further embodiment of the present invention, in un-stretched and stretched states;
Figures 5a and 5b are plan views of a pressure sensor device according to a further embodiment of the present invention, in un-stretched and stretched states;
Figures 6a and 6b are plan views of a pressure sensor device according to a further embodiment of the present invention, in un-stretched and stretched states;
Figure 7 is a schematic view of a pressure sensor device according to a further embodiment of the present invention;
Figure 8 is a semi-schematic side perspective view of an exemplary arrangement of sensor devices according to an embodiment of the present invention on a compression stocking worn by a human;
Figure 9 is a semi-schematic perspective view of a further exemplary arrangement of sensor devices according to an embodiment of the present invention on a compression sleeve in order to determine the volume of blood flow into a human organ;

Figure 10 is a perspective view of an electronic control and power supply unit for use with embodiments of the invention;

Figure 11 is a rear view of a further exemplary arrangement of a sensor device according to an embodiment of the present invention on a nappy in order to determine an indication of the fullness of the nappy; and

Figures 12a-d are cross-sectional front and plan views of a pressure sensor device according to a further embodiment of the present invention, in un-stretched and stretched states.

DETAILED DESCRIPTION

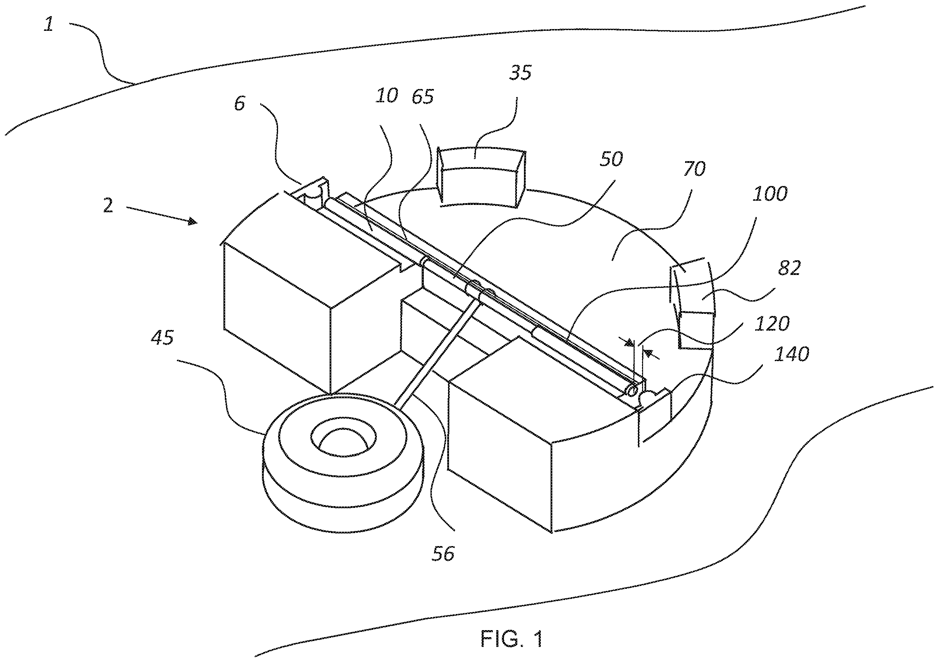

Figure 1 shows a compression garment apparatus according to an embodiment of the present invention. The apparatus comprises a compression garment 1 and a sensor device 2. The sensor device 2 comprises a plastic housing 70 and a stud 45. The plastic housing 70 and the stud 45 are affixed to different respective points (i.e. a first mounting point and a second mounting point respectively) on the outer surface of the compression garment 1 (i.e. the surface that faces away from the wearer when the garment is worn). The fastening may be achieved using hook-and-loop fastening pads, adhesive, stitching, riveting, or any other suitable mechanism.

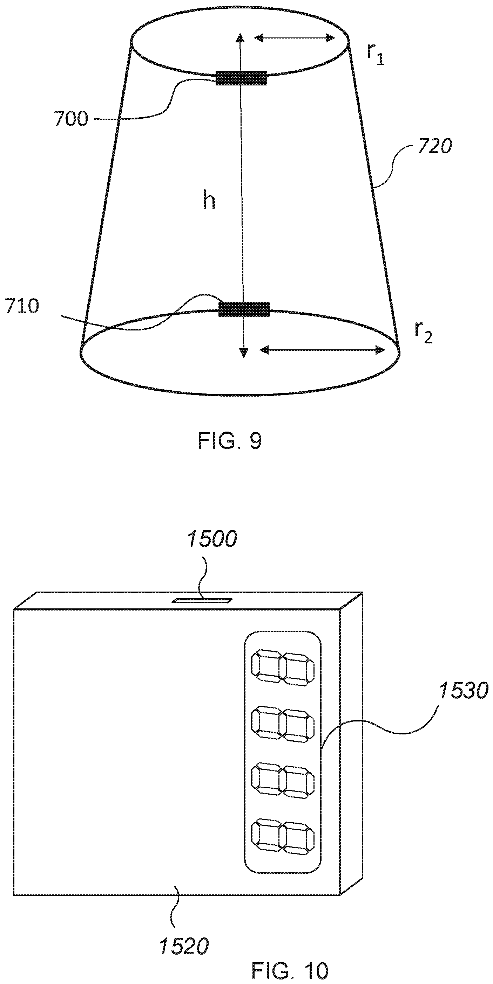

The apparatus also comprises an electronic controller (not shown), such as the electronic control and power supply unit described below with reference to Figure 10, which may be at least partly contained within the housing 70, or which may be separate from the housing 70 and stud 45 (e.g. connected by an electrical cable). A light-emitting diode (LED) 6 is mounted on the plastic housing 70, opposite a photodetector 140, which is also mounted on the plastic housing 70. A first rigid tube 10 and a second rigid tube 100 are mounted rigidly on the plastic housing 70, along respective portions of a path between the LED 6 and the photodetector 140. An optical fibre 50 extends through the first and second tubes 10, 100, with a first end adjacent the LED 6 and a second end adjacent the photodetector 140, so as to direct light produced by the LED 6 to the photodetector 140. The optical fibre has an outer diameter in the region of 0.25 mm - 1 mm. The first 10 and second tubes 100 each have an outer diameter of up to 2 mm and an inner diameter very slightly (e.g. between 0.05 mm and 0.2 mm) larger than the outer diameter of the optical fibre 50. The first 10 and second tubes 100 may be constructed from metal, ceramic, glass or plastic. The photodetector 140 is arranged to generate an electrical output signal corresponding to the amount of light it receives from the LED 6, through the optical fibre 50.

The first end of the optical fibre 50 is fixed to the front of the LED 6 (e.g. to a lens of the LED 6), while the second end of the optical fibre 50 is located at a distance 120 from the front of the photodetector 140 (e.g. spaced around 2 mm away). The second end of the optical fibre 50 is not fixed and is free to move longitudinally through the second tube 100. In other embodiments, the first end of the optical fibre 50 may instead be fixed adjacent the front of the photodetector 140 and the second end of the optical fibre 50 may be located adjacent the LED 6 but free to move longitudinally within the first tube 10.

A central portion of the optical fibre 50 is not contained within either the first tube 10 or the second tube 100. This exposed central region is approximately 10 mm in length. The first 10 and second tubes 100 are used to encase the optical fibre 50 and restrain it from lateral movement, apart from in the exposed central portion of the optical fibre 50.

A thread 56 comprises a first end and a second end. The first end of the thread 56 is attached to the stud 45 and the second end of the thread 56 is attached to the central portion of the optical fibre 50 that is not covered by either the first tube 10 or the second tube 100, approximately centrally between the first and second tubes 10, 100.

A device 2 further comprises a spring 65 having a first end and a second end. The first and second ends of the spring 65 are attached to the housing 70 adjacent the LED 6 and the photodetector 140 respectively, such that the spring 65 extends parallel to the optical fibre 50. The spring 65 is linked to the central portion of the optical fibre 50 by the second end of the thread 56.

The housing 70 further comprises locator studs 35, 82 for connecting a protective casing (not shown) around the optical fibre 50, LED 6, spring 65 and photodetector 140.

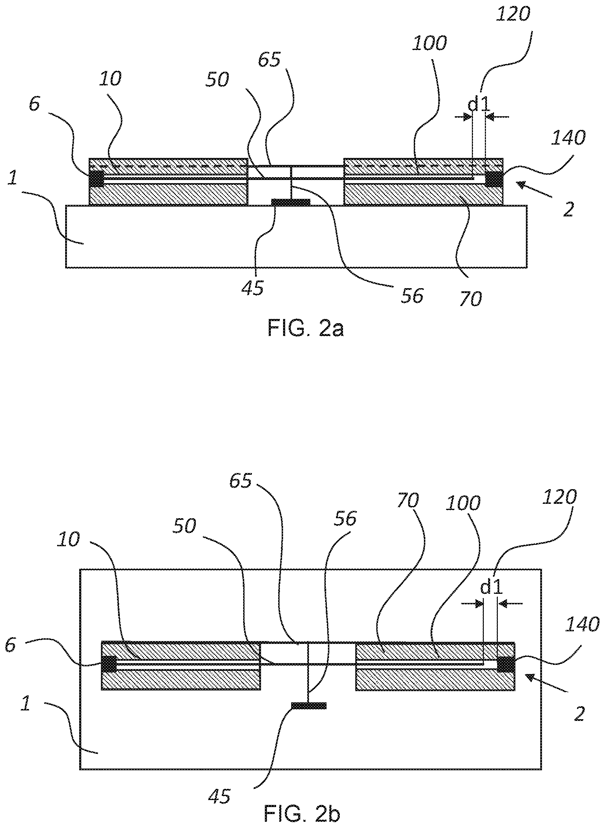

Figures 2a and 2b respectively show cross-sectional front and plan views of the device 2 of Figure 1 when the compression garment 1 is in a slack state-i.e. before stretching of the garment 1 over a patient's body.

In use, the stud 45 and housing 70 of the sensor device 2 are attached to the compression garment 1, with the thread 56 minimally taut, before the assembled compression garment apparatus is put on the wearer. In this state, the wire spring 65 is also minimally taut and thus exerts a zero or minimal biasing force on the thread 56 and the optical fibre 50. Typically, the stud 45 will be circumferentially displaced from the housing 70, so that the sensor measures circumferential displacement (e.g. displacement around a limb of the wearer). Before the garment 1 is applied, power is supplied to the device 2 (from an internal or external power supply such as a battery), causing light from the LED to pass through the optical fibre 50 to the photodetector 140, with the optical fibre 50 following a straight path.

The photodetector 140 produces an output signal that corresponds to an un-stretched state of the garment 1. The garment 1 is then applied to the wearer.

The garment 1 is stretched once it is put on the wearer, and so the distance between the stud 45 and the housing 70 increases, causing the thread 56 to pull the exposed central portion of the optical fibre 50 in the direction of the stud 45. This causes the distance 120 between the second end of the optical fibre 50 and the photodetector 140 to increase. This increase in distance 120 reduces the amount of light received by the photodetector 140 from the LED 6 via the optical fibre 50.The proportion of the emitted light that strikes the photodetector 140 reduces with increasing distance. Therefore, the electrical output signal produced by the photodetector 140 is lower once the garment 1 is fitted than when it was slack.

The movement of the stud 45 away from the housing 70 also causes the thread 56 to pull the central portion of the spring 65 in the direction of the stud 45, thus causing the spring 65 to extend. As a result of this extension, the spring 65 exerts a biasing force on the optical fibre 50 and the stud 45 via the thread 56. This biasing force acts against the tension in the thread 56, tending to restore the position of the stud 45 and the optical fibre 50 to the initial "un-stretched" position shown in Figures 2a and 2b. This biasing force helps to reduce the effects of hysteresis in the fibre when the garment is stretched and un-stretched repeatedly-e.g. when the garment 1 is repeatedly taken on and off.

If the stretch in the garment 1 is reduced, the separation between the stud 45 and the housing decreases, allowing the optical fibre 50 to extend towards the photodetector 140, thus reducing the distance 120 between the second end of the optical fibre 50 and the photodetector 140. This increases the amount of light received by the photodetector 140 from the LED 6 via the optical fibre. Therefore, the output signal produced by the photodetector 140 increases in this situation.

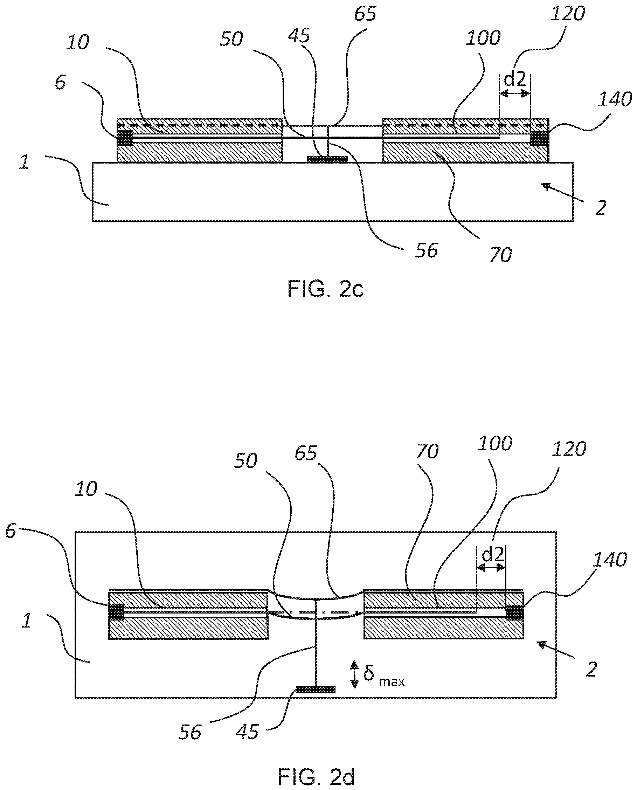

Figures 2c and 2d respectively show cross-sectional front and plan views of the device 2 with the garment 1 in a stretched state. As can be seen, the increase in the distance between the stud 45 and the housing 70 causes the thread 56 to pull on the exposed central portion of the optical fibre 50 and the central portion of the spring 65 such that the optical fibre 50 and the spring 65 deflect. The initial (un-stretched) position of the optical fibre 50 is shown by a semi-dashed line in Figured 2d. This deflection in the optical fibre 50 results in a change in the distance 120 between the second end of the optical fibre 50 and the photodetector 140, from $d$-, in a slack state, to a larger value, $d_2$, in the stretched state. As described above, while in the stretched state shown in Figures 2c and 2d, the spring 65 exerts a biasing force on the fibre 50 and the thread 56 that helps to restore the stud 45 and the fibre 50 to their original (un-stretched) positions once stretching of the garment is relaxed.

The change in distance 120 depends on the properties of the material from which the compression garment 1 is made, and on the properties of the optical fibre 50. The deflection of the optical fibre 50 in the exposed region in the direction of stretch is expressed analytically by the following equation:

$$6max = \frac{48E1}{(FL^3)} \qquad (1)$$

in which $o_{n,,,}$ is the deflection of the fibre 50 in the direction of the stretch, $F$ is the pulling force in the thread 56, $L$ is the length of the exposed central region of the optical fibre 50, $E$ is the Young's Modulus of the material of the optical fibre 50, and $I$ is the moment of inertia of the optical fibre 50.

When the garment 1 is stretched, the force $F$ (where the force of the spring 65 is considered in the force $F$) exerted on the optical fibre 50 by the thread 56 increases, thus increasing the deflection $o_{n,,,}$ of the fibre 50 according to equation (1). This deflection results in an increase in the distance $d_2$ between the second end of the optical fibre 50 and the photodetector 140. As the intensity of the light received by the photodetector 140 is proportional to $d_2$ the electrical output signal produced by the photodetector 140 decreases. Therefore, the electrical output of the photodetector 140 is proportional to the stretch distance $o_{n,,,}$ of the garment 1.

The electronic controller of the device 2 may, in some embodiments, accordingly map the output of the photodetector 140 to a value of stretch distance $o_{n,,,,}$, e.g. using a lookup table of empirically determined data stored in a memory of the controller.

The stretch distance $8_{n,,,}$ of the garment 1 is equal to the difference between the initial distance of the stud from the housing $x_1$ and the distance from the stud to the housing after the garment 1 has been stretched $x_2$. This difference is also proportional to the change in circumference of the garment 1 from an initial (un-stretched) circumference $C_1$ to a final (stretched) circumference C2, and to the tension $T$ in the garment 1 (as shown by the factor $F$ in equation (1) above and by $T$ in equation (2) below).

The pressure exerted by the compression garment 1 on the body is determined by the well-known Laplace Law:

$$P = T/r \qquad (2)$$

in which $P$ is the pressure, $T$ is the tension in the garment 1 material, and $r$ is the radius of curvature of the garment. However, the circumference of the garment 1 may be used instead of the radius of curvature. As the tension $T$ is proportional to the stretch distance of the garment 1, the tension $T$ may be determined from the output of the photodetector 140.

The controller connected to the device 2 may, in some embodiments, map the output of the photodetector 140 to a value of tension *T,* e.g. using a lookup table of empirically determined data.

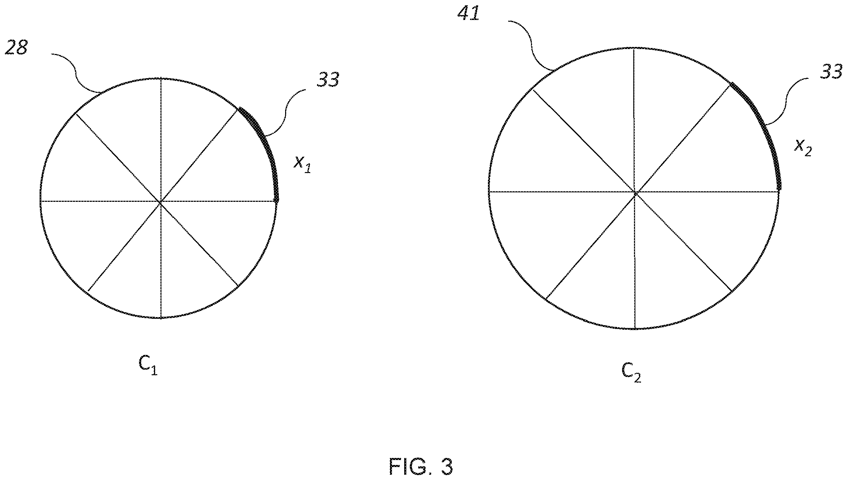

The method for determining an accurate value of compression pressure from the measurements obtained by the sensor device is explained below with reference to Figure 3.

The pressure can be calculated by the electronic controller-e.g. using software executed by a processor, or using custom hardware logic such as an FPGA or ASIC. A data logger (not shown) can then record the values of the pressure at intervals. Data may be logged as frequently as required and either displayed in real-time or retrieved via a USB port 1500 (shown in Figure 10).

Figure 3 schematically represents the change in circumference of the compression garment when stretched. The un-stretched garment is represented by a circle 28 and the stretched garment is represented by a larger circle 41. The initial and final circumferences of the garment, before stretching and after stretching, are Ci and C2 respectively. The initial circumference Ci of the garment is typically specified by the manufacturer of the garment. It can be stored as a constant by the controller.

The circumference $C_1$, $C_2$ of each circle 28, 41 has been equally divided into eight arcs. The difference between the length xi of an arc 33 of the circle 28 prior to stretching and the length $x_2$ of the same arc 33 of the circle 41 after stretching is given by $Ax = x_2 - x_1$. Thus, the arc 33 corresponds to a respective displacement between the stud 45 and the plastic housing 70. It will be appreciated that Ax may correspond to an increase or a reduction in distance, depending on whether the amount of stretching of the garment is increasing or decreasing.

If $N_1 = C_1/x_1$, where $N_1$ is the number of arcs, and $N_1 = N_2 = C_2/X_2$, then $C_1/x_1 = C_2/x_2$. This may be rearranged to $C_2 = {}^{c}ix2$ .

Furthermore, $Ax = x_2 - x_1$ may be rearranged to give $x_2 = Ax +$

Thus:

$$\frac{C_i(Ax + \underline{x1})}{X_i} C_2—$$

$$C_2 = \frac{C_iAx}{x_i} + C_i$$

$$C_2 = C_1 + {}^{'}fi_x$$

$$C_2 = \frac{}{x_i} —Ax$$

$$C_2 = Aix,$$

where *A* is a constant defined by $A = -$, which may be pre-stored in a memory of the controller.

Consequently, the pressure applied by the garment when stretched is given by the following equation:

$$P = (^{3}) c_i + AAx$$

Both T and Ax are proportional to the amount of stretch and may be determined from the change in the output signal produced by the photodetector 140 when the garment is stretched. As the change in change in arc length Ax is likely to be very small in comparison to the radius of the garment, Ax may be assumed to be equal to the linear displacement $o_{max}$ of the stud from the housing, as discussed above.

Thus, in order to calculate the pressure *P* applied by the garment, the controller may calculate a value of tension T in the garment, and a value of the linear displacement $o_{n,,,} = /x$ of the stud 45 from the housing 70, from the change in the output signal produced by the photodetector 140, e.g. using a lookup table. The controller then calculates the pressure *P* as the determined tension T divided by a linear function of the determined displacement ,Ax. It preferably does this by numerically evaluating a function of T and /x (e.g. using floating point arithmetic operations), but it could determine an approximate pressure estimate by using a multi-dimensional look-up table of pre-calculated pressures for sets of possible values for T and ,Ax.

In some embodiments the apparatus may determine a qualitative measure (e.g. a binary determination of whether the garment is sufficiently stretched or not). The controller may compare the calculated pressure estimate against a threshold value, or it could directly compare the voltage output of the photodetector 140 with a predetermined threshold value, to detect when it passes below the threshold, as being indicative of having sufficient tension in the garment. This may be used to provide an indication of whether or not the garment is being (or has been) worn. In other embodiments, (instead of, or as well as, a voltage input), a capacitance, resistance, magnetic field strength or other electrical or optical signal as appropriate may be compared to a predetermined threshold value to determine whether there is sufficient tension in the garment.

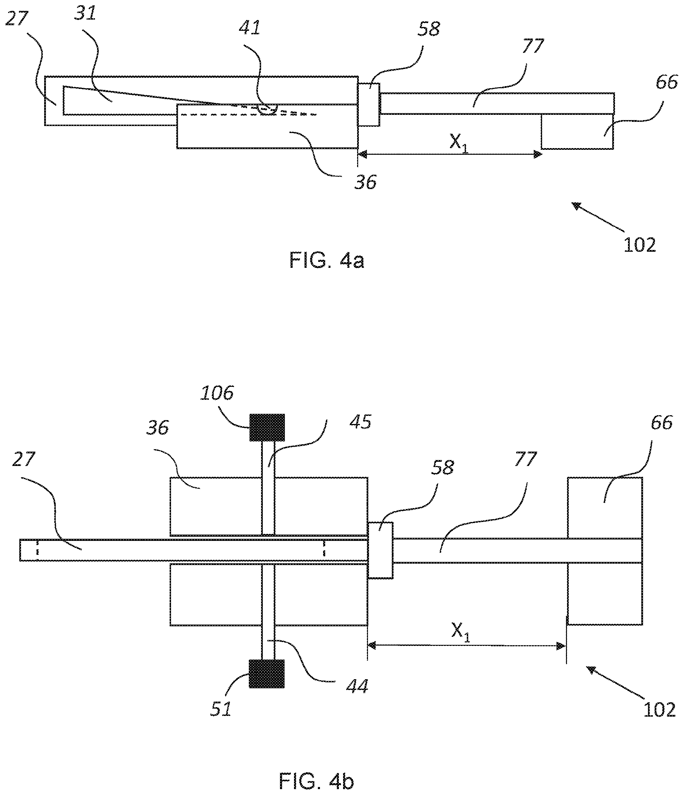

Figures 4a and 4b respectively show cross-sectional front and plan views of a sensor device 102 in accordance with an alternative embodiment of the present invention, in which an obscuring element 31 is moved by the stretching of a compression garment (not shown) to interrupt an optical beam.

In this embodiment, a housing 36 retains a first optical fibre 45, which receives light at a first end from an LED 106, and a second optical fibre 44, arranged coaxially with the first optical fibre 45 such that it receives light, at first end, from a second end of the first optical fibre 45 and outputs the light from a second end to a photodetector 51. An elongate arm 27 is arranged between the second end of the first optical fibre 45 and the first end of the second optical fibre 44. The arm 27 is perpendicular to the shared axis of the first 45 and second optical fibres 44. The arm 27 is arranged to move longitudinally relative to the housing 36. The arm 27 comprises an obscuring element 31, which has a substantially triangular, wedge-shaped cross-section, an end-stop 58 and an extended portion 77. The distal end of the extended portion 77 is fixedly mounted on an anchor block 66.

The anchor block 66 and the housing 36 are affixed to respective points of the outer surface of a garment, around a circumference of the garment. Prior to the stretching of the garment, they are separated by a distance $x_1$.

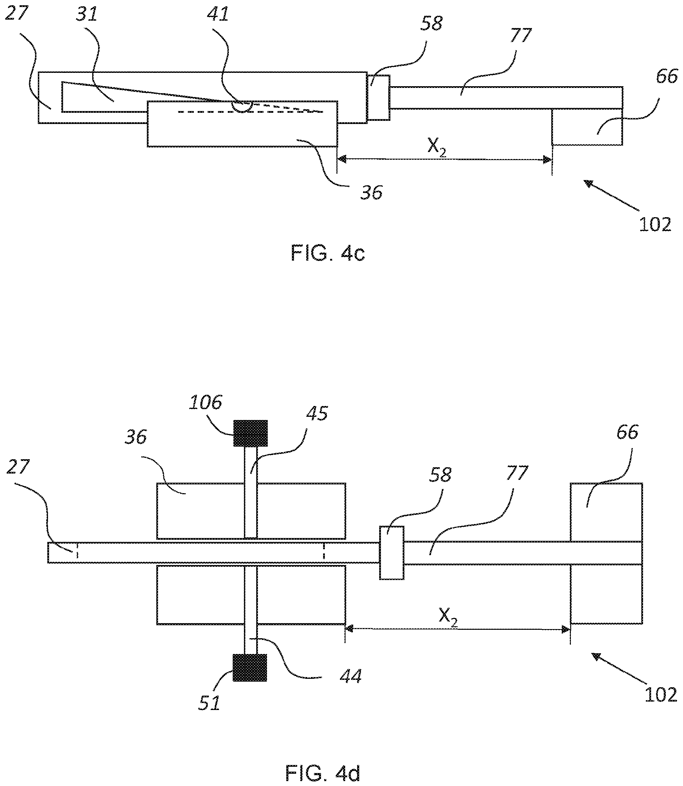

The housing 36 further comprises a void 41 through which light from the first optical fibre 45 is directed into the second optical fibre 44. The arm 31 is movable relative to the housing 36 between an un-stretched position (shown in Figures 4a and 4b) and a stretched position (shown in Figures 4c and 4d).

In the un-stretched position, the obscuring element 31 is arranged to interrupt the beam of light as it passes through the void 41 between the first 45 and second optical fibres 44 to small degree, or not at all. Thus, in this position, the photodetector 51 receives a maximum amount of light from the LED 106.

Figures 4c and 4d respectively show cross-sectional front and plan views respectively of the device 102 of Figures 4a and 4b with the garment in the stretched position. The stretch in the garment causes the anchor block 66 and the housing 36 to move apart by a distance $-max = x_2 - x_2$ such that the anchor block 66 and the housing 36 are separated by a distance $x_2$, as shown in Figures 4c and 4d.

In the stretched state, the obscuring element 31 is arranged to interrupt the beam of light between the first 45 and second optical fibres 44 to a greater extent, due to its wedge shape. Thus, in this position, the photodetector 51 receives a lesser amount of light from the LED 106. The photodetector 51 is arranged to produce an electrical output signal that is proportional to the amount of light received from the LED 106.

A high resolution analogue signal is produced by the photodetector 51. This signal may optionally be converted to a digital signal by an analogue-to-digital converter for display and/or data logging purposes.

The change in output signal from the photodetector 51 corresponds to the change in circumference of the garment. Therefore, the compression pressure may be determined, and optionally logged by an electronic controller, in a similar way to that described above.

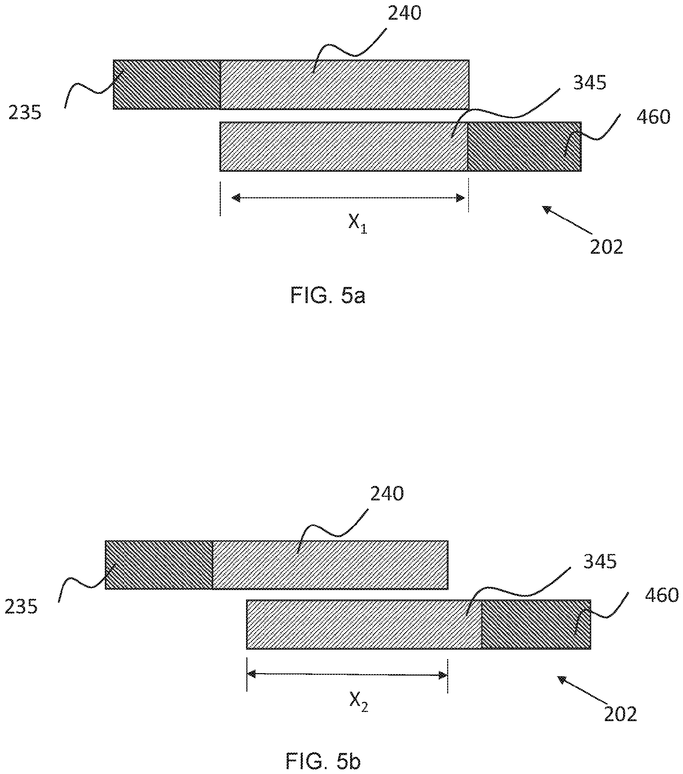

Figures 5a and 5b show cross-sectional views of a pressure sensor device 202 according to an alternative embodiment of the present invention in which an organic photodetector 345 is arranged to detect light emitted from an organic LED 240.

Organic devices have a very low profile, which is convenient for applications where the devices are arranged on a garment. Furthermore, such devices are inexpensive to manufacture in large quantities.

In this embodiment, a first end 235 of an elongate organic LED 240 can be fixed to a first region of a compression garment (not shown), and a first end 460 of an elongate organic photodetector 345 can be fixed to a second region of the compression garment, such that the LED 240 and the photodetector 345 are in a side-by-side overlapping arrangement, both oriented parallel with a circumferential direction of the compression garment, and with first ends 235, 460 distal from each other. Stretching of the compression garment thus causes the first end 235 of the organic LED 240 and the first end 460 of the organic photodetector 345 to move apart, reducing the length of overlap.

The LED 240 is arranged such that an elongate light-emitting portion of the LED 240 is alongside an elongate light-detecting portion of the photodetector 345. Thus, light emitted from this portion of the LED 240 can be received by the photodetector 345. Light emitted from portions of the LED 240 that are not overlapping the light-detecting portion of the photodetector 345 is not detected by the photodetector 345.

The photodetector 345 is arranged to produce an output signal that is proportional to the amount of light received by the photodetector 345 from the LED 240. As with the other embodiments herein, the sensor device may comprising a casing (not shown) which may shield the photodetector 345 from ambient light.

Figure 5a shows the arrangement of the device 202 before the garment is stretched. As can be seen, a region of the LED 240 of initial length $x_1$ is opposite the photodetector 345. Thus, in Figure 5a, the photodetector 345 receives incident light from this portion of the LED 240 alone and produces a corresponding output signal.

Figure 5b shows the arrangement of the device 202 during stretching of the garment. As can be seen, the region of the LED 240 that is opposite the photodetector 345 has decreased in length to $x_2$. Thus, the amount of light received by the photodetector 345 from the LED 240 is reduced, in proportion with the displacement of the two ends 235, 460, which means that the photodetector 345 produces a lower output signal.

Thus, the change in output signal corresponds to a change in circumference of the garment and may therefore be used to determine a compression pressure, as disclosed above.

In alternative embodiments, the optical elements may be replaced by an inductive or magnetic sensor having appropriate fixed and movable parts where the inductive and magnetic sensing elements may be of a small footprint MEMS (micro electromechanical systems) type.

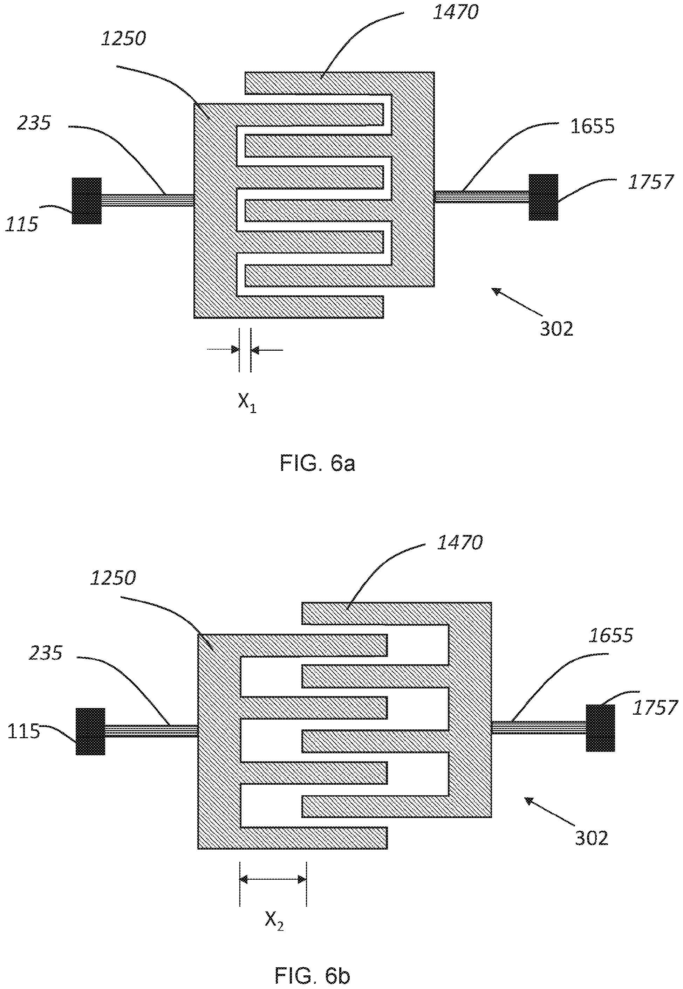

Figures 6a and 6b show plan views of a pressure sensor device 302 according to an alternative embodiment of the present invention in which a variable capacitor is used to determine the change in circumference of a compression garment after stretching.

Figure 6a shows a capacitor comprising a first plate 1250 and a second plate 1470. The capacitor may be a thin film MEMS-type micro-fabricated capacitor. As can be seen in Figure 6a, the first 1250 and second capacitor plates 1470 comprise a number of interdigitated 'fingers', which may be up to 0.2 mm wide. The capacitor plates 1250, 1470 may be around 0.1 mm in thickness and are separated by an initial (un-stretched) distance $x_1$ of up to 0.1 mm.

Capacitor plates 1250, 1470 are connected to electronic circuitry (not shown) via arms 235, 1655 that terminate at mounting pads 115, 1757. The electronic circuitry is configured to measure the capacitance of the capacitor. The mounting pads 115, 1757 may be constructed from thin films (which may be deposited, screen printed etc.). Each of the mounting pads 115, 1757 is mounted on a structure (not shown) that can be affixed to the outer surface of a compression garment, e.g. by a detachable hook-and-loop connecting mechanism.

Figure 6b shows the device 302 of Figure 6a after stretching of the garment. As can be seen, the stretching of the garment causes the mounting pads 115, 1757 to move apart, thus causing the capacitor plates 1250, 1470 to move apart such that the distance between the capacitor plates 1250, 1470 increases to $x_2$.

The increase in distance between the capacitor plates 1250, 1470 reduces the capacitance of the capacitor that is measured by the electronic circuitry. The capacitance will change in some proportion to the change in circumference of the garment caused by stretching. Therefore, a correlation may be determined between the change in capacitance and the change in circumference of the garment during stretching. Thus, Equation 2 may be used to calculate a compression pressure from a determined change in circumference of the garment, if required.

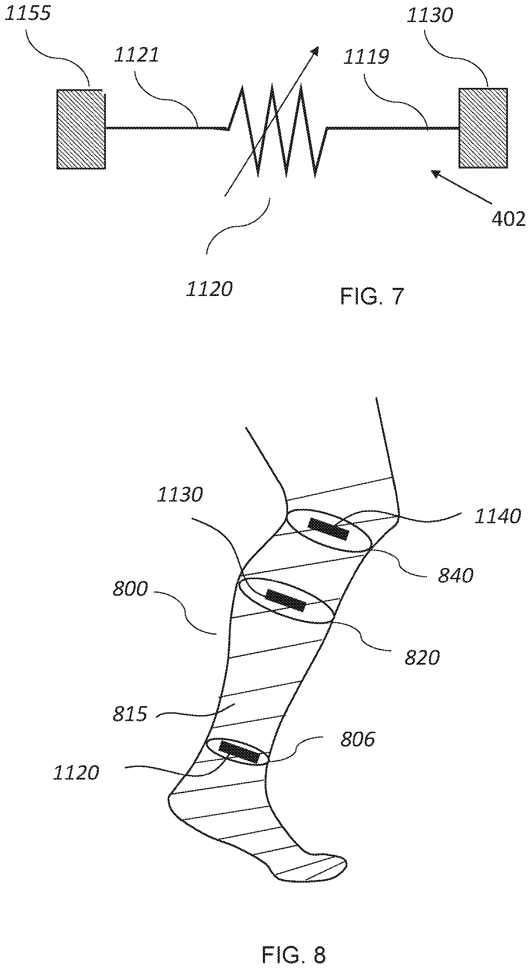

Figure 7 is a schematic view of a sensor device 402 according to an alternative embodiment of the present invention in which a variable resistor 1120 is used to determine the change in circumference of a compression garment.

The device 402 comprises a first 1155 and second mounting pad 1130 that can be affixed to the outer surface of a compression garment, typically along a circumferential path. The device 402 further comprises a variable resistor 1120, the resistance of which is arranged to vary according to the application of a mechanical load. The variable resistor 1120 may be a strain gauge, conductive polymer, piezoresistive element or similar. The variable resistor 1120 is fixedly connected to the first 1155 and second 1130 mounting pads by a first 1121 and second wire 1119 respectively.

The variable resistor 1120 is electrically connected to an electronic circuit (e.g. comprising a Wheatstone-Bridge circuit) (not shown) that is arranged to measure the resistance of the variable resistor 1120. The variable resistor 1120 may comprise an inductor, piezoelectric element or similar, that is connected to the electronic circuit such that the strain on the sensing element during stretching of the garment produces a change in the output signal.

During stretching of the garment, the first mounting pad 1155 is moved away from the second mounting pad 1130. As a result, the first 1121 and second wires 1130 exert a tension on the variable resistor 1120 such that the resistance of the variable resistor 1120 changes. The change in resistance of the variable resistor 1120 is proportional to the tension exerted by the first 1121 and second wires 1130 and is therefore proportional to the amount by which the garment is stretched. Consequently, a change in circumference of the garment may be determined and subsequently used to calculate a compression pressure according to Equation 2.

Figure 8 shows an exemplary arrangement of three sensor devices, each embodying the invention, positioned on a compression stocking 815 worn on a leg 800 of a patient. These may communicate with one or more controllers in one or more of the sensor devices, or with a controller that is remote from the sensor devices. The sensor devices and controller(s) may embody a sensor apparatus as disclosed herein.

Compression stockings 815 have several uses including the management of venous leg ulcers. The compression applied to the leg 800 may be 'graduated', meaning that the compression pressure is higher (e.g. 40 mm Hg) at the ankle and is gradually decreased towards the knee (e.g. to 15 mm Hg). If the compression pressure is too high, it may cause problems with the patient's skin. On the other hand, if the compression pressure is too low, the effectiveness of the compression treatment is reduced. Correct application of compression allows faster healing. Therefore, it is important for clinicians to know the amount of compression applied.

In this example, before the compression stocking 815 is put on the leg 800 of the patient, sensor devices 1120, 1130, 1140 are positioned on the outer surface of the compression stocking 815 at various locations along the length of the stocking 815. The first sensor device 1120 is positioned just above the ankle 806. The second sensor device 1130 is positioned at the mid-calf 820 area. The third sensor device 1140 is positioned just below the knee 840.

The sensor devices 1120, 1130, 1140 may be used to determine the distribution of pressure over the compression garment 815. The data generated by the sensor devices 1120, 1130, 1140 may then be analysed to establish whether then compression garment is performing as required.

Figure 9 shows an exemplary arrangement of sensor devices according to any embodiment of the present invention when positioned on a compression sleeve 720.

In some circumstances, it may be desirable to measure the swelling of a part of the body. For example, this is required in case of patients suffering from lymphedema. Compression sleeves are used after a surgical procedure to remove trapped fluid from the lymphatic system and reduce swelling. The decrease in swelling in the affected area is used as an indication of the patient's improvement.

As a further example, athletes may wish to measure the volume change in a body part when performing certain exercises, e.g. to determine muscle growth. The volume change may be related to a change in blood flow to or from the body part or a change in muscle volume.

Figure 9 shows an exemplary compression garment 720 that may be worn on, for example, an arm or leg. The compression garment 720 has an upper radius $r_1$, measured at a distal end of the compression garment 720, a lower radius $r_2$, measured at a proximal end of the compression garment 720, and a height $h$.

The volume $(V)$ contained within the compression garment 720 may be determined according to the equation:

$$V = \tfrac{\pi}{3}(7^-{}_1{}^2 + r_i r_2 + r_2{}^2)h \qquad\qquad (4)$$

A first sensor device 710 is located on the surface of the distal end of the compression garment 720 (e.g. located just below the knee of the wearer). A second sensor device 700 is located on the surface of the proximal end of the compression garment 720 (e.g. located around the ankle of the wearer).

A change in the lower radius $r_2$ and the upper radius $r_l$ caused by a stretching of the compression garment 720 may be determined according to any of the methods discussed above. Thus, Equation 4 may be used to determine a change in volume of the compression garment 720 that corresponds to a change in volume of the body part around which the compression garment 720 is worn.

For higher accuracy, additional sensor devices may be located at an intermediate locations (e.g. at the mid-calf area). In this case, the above equation would be used multiple times and the results summed to determine the total volume.

Where multiple sensor devices are used (e.g. between two and twenty) on a single compression garment, the sensor devices may be linked electronically to facilitate the construction of a map of the pressure distribution. The sensor devices may be distributed linearly along the length of the compression garment or around a circumference. Each sensor device may be accessed individually or simultaneously to detect pressure distribution.

In the case of a muscular volume change, it may also be possible to determine a blood flow rate from the change in volume of the muscle, as the change in volume of a muscle is related to the rate of blood flow in the leg.

Figure 10 shows an electronic control and power supply unit 1520 (i.e. a controller) that may be used with any embodiment of the invention. The control and power supply unit 1520 may comprise signal amplifiers, an analogue-to-digital converter, a microcontroller or other processor, memory, a data logger, a wireless transmission (e.g. Bluetooth™) system, discrete or integrated electronic components, FPGAs, DSPs, ASICs, etc. It may have a digital display 1530 which is configured to display instantaneous pressure readings. The electronic control and power supply unit may comprise an audio indicator-e.g. for signalling an alert if the pressure applied by the compression garment is outside a target range-e.g. if the garment is pressing too loosely and/or is pressing too tightly. The entire electronic control and power supply may be contained within a housing of the unit 1520.

Measurements determined by the sensor device can be captured at intervals and stored in the data logger prior to processing in the microprocessor. Measurements may be taken and recorded in the data logger as frequently as desired-e.g. every minute or every hour. Data from the data logger or the microprocessor may be transmitted by the transmission system to a remote device or server. This may be a smartphone, laptop, pager or other device. The remote device can keep a record of pressure data that can be used for clinical evaluation or performance assessment by athletes.

This data logging may be used by clinicians to monitor the healing response of an individual under different compression conditions. However, such logging is not essential, and the control unit 1530 may simply provide an instantaneous indication of displacement of the compression garment.

The control and power supply unit 1520 further comprises a rechargeable coin cell for supplying electrical power to the claimed pressure sensor device and the electrical components of the control and power supply unit 1520. The power supply unit 1520 supplies a voltage of 5V or less. The control and power supply unit 1520 further comprises a USB port 1500 by which data may be retrieved from the data logger.

The control and power supply unit 1520 may be attached to a garment via adhesive pads, a hook-and-loop connecting mechanism or similar, or it may be integrated into a housing or casing of the sensor device, or it could be connected by a cable and located remotely-e.g. in a pocket of clothing worn by the wearer, or on a bedside trolley.

Although the above embodiments describe the sensor device of the present invention with regard to uses on a compression garment, where compression forces are relevant, any features of the above-described embodiments can also be applied to embodiments that deduce the expansion of a garment more generally, e.g. expansion of a nappy.

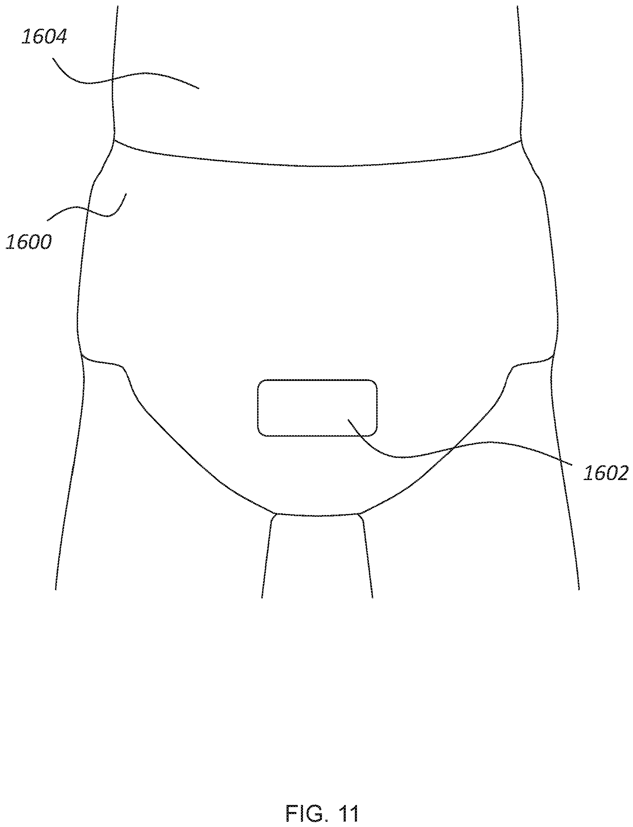

Figure 11 shows a sensor device 1602 in accordance with an embodiment of the present invention, attached to the outer surface of a nappy 1600 (diaper) that is being worn by a baby 1604. It will be appreciated that the nappy could instead be worn by an adult.

The first and second mounting points (not shown) of the sensor device 1602 are attached to respective first and second points on an outer surface of the nappy 1600. These points may be any appropriate distance apart-e.g. around 2 cm apart when the nappy is dry and unsoiled.

A first displacement between the first and second mounting points is measured before or soon after the nappy 1600 has been put onto the baby 1604, or is preconfigured. This first displacement is representative of the state or stretch in the nappy 1600 when the nappy 1600 is in an unsoiled 'empty' state.

Subsequently, the sensor device 1602 records, at intervals, further measurements of the displacement between the first and second mounting points of the sensor device 1602. Each further measurement of displacement can be compared with the first displacement. If the device 1602 records a displacement measurement that is greater than the first displacement by a predetermined threshold, indicating a significant expansion of the nappy 1602, the device 1602 determines that the nappy 1602 has been filled, e.g. by urine or faeces. Consequently, the device 1602 may be arranged to issue an alert to the parent or guardian of the baby 1604 that the nappy 1600 has been soiled and therefore requires changing-e.g. by sending a radio message to a baby monitor device, or to an app on a phone belonging to the parent or guardian. In some embodiments, more complex processing of the displacement measurement may be performed, e.g. to filter out changes arising due to movement of the baby (e.g. crawling).

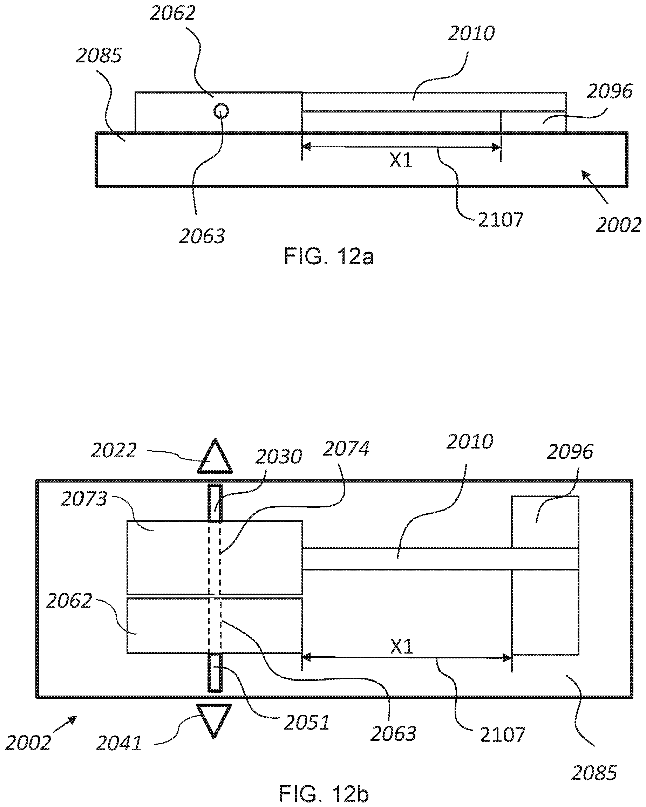

Figures 12a and 12b respectively show cross-sectional front and plan views of a sensor device 2002 in accordance with an alternative embodiment of the present invention, in which a light emitting diode (LED) 2041 is connected to a first optical fibre 2051, and a photodetector 2022, connected to a second optical fibre 2030, is arranged to detect light emitted from the LED 2041 through the first optical fibre 2051.

In this embodiment, the sensor device 2002 comprises a first anchor block 2062 and a second anchor block 2096 that are attached to respective first and second mounting points on an outer surface of a garment 2085, such as a compression stocking or a nappy (diaper), around a circumference of the garment 2085. A rigid elongate arm 2010 is arranged to connect the second anchor block 2096 to a third anchor block 2073 so as to maintain a fixed displacement between the second anchor block 2096 and the third anchor block 2073. The third anchor block 2073 lies adjacent the outer surface of the garment 2085 such that the third anchor block 2073 and the first anchor block 2062 are adjacent. The third anchor block 2073 is not fixedly mounted on the garment 2085. Thus, displacement of the second anchor block 2096 relative to the first anchor block 2062 (e.g. owing to stretching the garment 2085) results in a corresponding displacement of the third anchor block 2073 relative to the first anchor block 2062.

The first anchor block 2062 defines a first through-bore 2063 that extends from an LED 2041, mounted on the first anchor block 2062, through the first anchor block 2062. The third anchor block 2073 defines a second through-bore 2074 that extends from a photodetector 2022, mounted on the third anchor block, through the third anchor block 2073. The first anchor block 2062 and the third anchor block 2073 are mounted adjacent one another on the garment 2085 such that, prior to stretching of the garment 2085, the first through-bore 2063 and the second through-bore 2074 are coaxial (i.e. they extend along the same axis).

The sensor device 2002 comprises a first optical fibre 2051, retained within the first through-bore 2063, and a second optical fibre 2030, retained within the second through-bore so as to be in alignment with the first optical fibre 2051 in an un-stretched state of the garment 2085. A first end of the first optical fibre 2051 receives light from the LED 2041. A first end of the second optical fibre 2030 is arranged to receive light (from the LED 2041) from a second end of the first optical fibre 2051. The photodetector 2022 is arranged to detect light transmitted through the second optical fibre 2030. Thus, the photodetector 2022 is arranged to detect, via the first optical fibre 2051 and the second optical fibre 2030, the light emitted by the LED 2041.

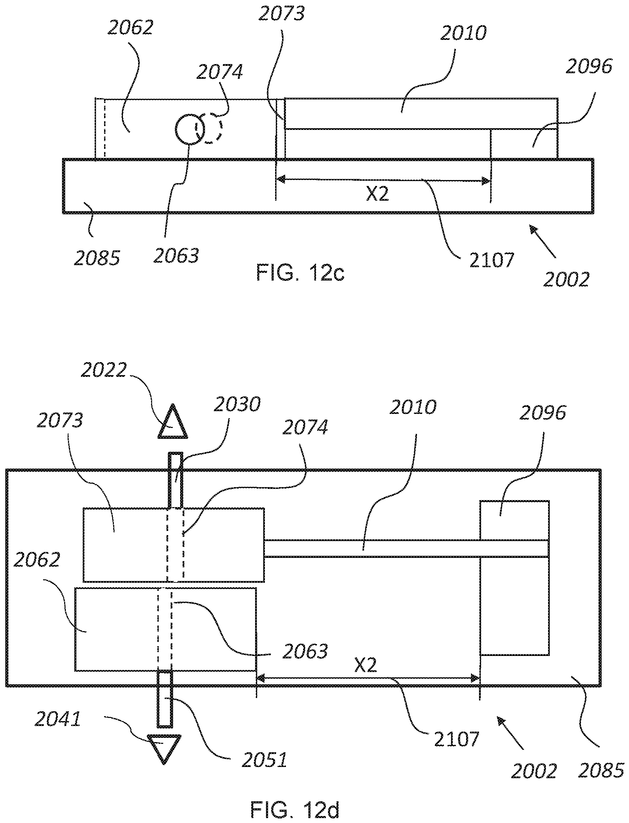

Prior to stretching of the garment 2085, the first anchor block 2062 and the second anchor block 2096 are separated by a distance 2107 ($x_1$), in a direction perpendicular to the axis along which the first optical fibre 2051 extends through the first anchor block 2062. When the garment 2085 is stretched (e.g. when it is being worn), the distance 2107 between the first anchor block 2062 and the second anchor block 2096 increases from $x_1$ to $x_2$. This is shown in Figures 12c and 12d.

The displacement between the first anchor block 2062 and the second anchor block 2096 results in a corresponding displacement between the first anchor block 2062 and the third anchor block 2073 and, thus, a misalignment of the first optical fibre 2051 and the second optical fibre 2030. The misalignment in the fibres 2051, 2030 means that cross-sectional area of the second optical fibre 2030 that is exposed to the cross-sectional area of the first optical fibre 2051 (and, thus, the light transmitted through the first optical fibre 2051 to the second optical fibre 2030) is reduced. As a result, the photodetector 2022 measures a decrease in the received light intensity.

The photodetector 2022 is arranged to produce an electrical output signal that is proportional to the amount of light received from the LED 2041. A high resolution analogue signal is produced by the photodetector 2022. This signal may optionally be converted to a digital signal by an analogue-to-digital converter for processing by a controller as disclosed herein. In particular, the change in output signal from the photodetector 2022 corresponds to the change in circumference of the garment 2085; therefore, the compression pressure exerted by the garment 2085 can be determined, and optionally logged by an electronic controller, in ways described herein.

It will be appreciated by those skilled in the art that the invention has been illustrated by describing one or more specific embodiments thereof, but is not limited to these embodiments; many variations and modifications are possible, within the scope of the accompanying claims.

**Claims**

1. A sensor apparatus comprising a sensor device (2; 102; 202; 302; 402; 710; 1602; 2002), for attaching to an outer surface of a compression garment (1; 720), and a controller, wherein the sensor device comprises:

   a first mounting point for attaching to a first point on the outer surface of the compression garment; and
   a second mounting point for attaching to a second point on the outer surface of the compression garment, wherein the sensor device is arranged to sense displacement between the first and second mounting points; and wherein the controller is configured to process information representative of the sensed displacement to estimate a pressure exerted by the compression garment on a wearer of the compression garment.

2. The sensor apparatus of claim 1, wherein the sensor device (2; 102; 202; 302; 402; 710; 720; 1602; 2002) is arranged for non-destructively detachable attachment to the compression garment.

3. The sensor apparatus of any of claims 1 or 2, wherein the sensor device (2; 102; 202; 710; 1602; 2002) further comprises a light-emitting component (6; 106; 240; 2041) and a light-receiving transducer (140; 51; 345; 2022) and the sensor device is configured so that increased displacement between the first and second mounting points causes less light to strike the light-receiving transducer, resulting in a lower output signal from the transducer.

4. The sensor apparatus of claim 3, wherein the sensor device (2; 102) comprises a light-interrupting element (31) configured to progressively occlude an optical path between the light-emitting component (106) and light-receiving transducer (51) in dependence on the displacement between the first and second mounting points.

5. The sensor apparatus of any of claims 3 to 4, wherein the sensor device (2; 102; 2002) further comprises an optical fibre (50) extending at least partly between the light-emitting component (6) and the light-receiving transducer (140).

6. The sensor apparatus of claim 5, wherein the second mounting point is coupled to a point on the optical fibre (50) such that the movement of the second mounting point relative to the first mounting point causes the optical fibre to bend so that opposite ends of the optical fibre are drawn closer together or are pushed further apart.

7. The sensor apparatus of claim 5 wherein the sensor device (102; 2002) comprises a first optical fibre (45; 2051) and a second optical fibre (44; 2030), wherein the first optical fibre is arranged to receive light emitted from the light-emitting component (106; 2041) and to transmit the light to the second optical fibre, and wherein the sensor device is configured so that increased displacement between the first and second mounting points causes a relative lateral movement between a light-emitting end of the first optical fibre and a light-receiving end of the second optical fibre such that less light is transmitted from the first optical fibre to the second optical fibre.

8. The sensor apparatus of any preceding claim, wherein the sensor device (302) further comprises a first capacitor plate (1250) coupled to the first point, and a second capacitor plate (1470) coupled to the second point, for providing a variable capacitance between the first and second capacitor plates, wherein the sensor device is configured to use the variable capacitance between the first and second capacitor plates to sense displacement between the first and second mounting points.

9. The sensor apparatus of any preceding claim, wherein the sensor device (302; 402) is configured to use a variable resistance, or a variable magnetic field, or a variable electric field, to sense displacement between the first and second mounting points.

10. The sensor apparatus of any preceding claim, comprising a biasing member (65) arranged to resist a displacement between the first mounting point and the second mounting point.

11. The sensor apparatus of any preceding claim, wherein the controller is configured to calculate an estimated pressure exerted by the compression garment on a wearer of the compression garment by:

    determining a relative or absolute displacement value from the information representative of the sensed displacement;
    determining a relative or absolute tension force value from the information representative of the sensed displacement;
    calculating the estimated pressure by dividing the determined tension force value by a linear function of the determined displacement value.

12. The sensor apparatus of claim 11, wherein the linear function is the sum of a first constant and a product, wherein the product is the product of a second constant and the determined displacement value, and wherein the first constant is equal to the circumference of the compression garment in an un-stretched state.

13. A compression garment apparatus comprising a compression garment (1; 720) and a sensor apparatus as claimed in any preceding claim, wherein the sensor device (2; 102; 202; 302; 402; 710; 1602; 2002) of the sensor apparatus is attached to an outer surface of the compression garment.

14. The compression garment apparatus of claim 13, wherein the compression garment comprises a plurality of layers and the sensor device is attached to an outer surface of one layer of the compression garment.

15. The compression garment apparatus of any of claims 13 to 14, comprising one or more further sensor devices, each further sensor device comprising:

    a respective first mounting point for attaching to a respective first point on the outer surface of the compression garment; and
    a respective second mounting point for attaching to a respective second point on the outer surface of the compression garment,
    wherein each further sensor device is arranged to sense displacement between the respective first and second mounting points;
    and wherein the controller is configured to determine a rate of blood flow or a change in muscle volume from the respective sensed displacements.

**Patentansprüche**

1. Sensoreinrichtung, umfassend eine Sensorvorrichtung (2; 102; 202; 302; 402; 710; 1602; 2002) zum Anbringen an einer Außenseite eines Kompressionskleidungsstücks (1; 720) und eine Steuerungseinrichtung, wobei die Sensorvorrichtung umfasst:

    einen ersten Befestigungspunkt zum Anbringen an einem ersten Punkt auf der Außenseite des Kompressionskleidungsstücks; und
    einen zweiten Befestigungspunkt zum Anbringen an einem zweiten Punkt auf der Außenseite des Kompressionskleidungsstücks,
    wobei die Sensoreinrichtung dafür eingerichtet ist, eine Verschiebung zwischen dem ersten und dem zweiten Befestigungspunkt zu erfassen; und
    wobei die Steuerungseinrichtung dafür konfiguriert ist, Informationen zu verarbeiten, welche die erfasste Verschiebung darstellen, um einen Druck abzuschätzen, der durch das Kompressionskleidungsstück auf einen Träger des Kompressionskleidungsstücks ausgeübt wird.

2. Sensoreinrichtung nach Anspruch 1, wobei die Sensorvorrichtung (2; 102; 202; 302; 402; 710; 720; 1602; 2002) zur zerstörungsfrei abnehmbaren Anbringung am Kompressionskleidungsstück eingerichtet ist.

3. Sensoreinrichtung nach einem der Ansprüche 1 oder 2, wobei die Sensorvorrichtung (2; 102; 202; 710; 1602; 2002) ferner eine lichtemittierende Komponente (6; 106; 240; 2041) und einen lichtempfangenden Messwandler (140; 51; 345; 2022) umfasst und die Sensorvorrichtung so konfiguriert ist, dass eine erhöhte Verschiebung zwischen dem ersten und dem zweiten Befestigungspunkt bewirkt, dass weniger Licht auf den lichtempfangenden Messwandler trifft, was zu einem niedrigeren Ausgangssignal vom Messwandler führt.

4. Sensoreinrichtung nach Anspruch 3, wobei die Sensorvorrichtung (2; 102) ein lichtunterbrechendes Element (31) umfasst, das dafür konfiguriert ist, einen Strahlengang zwischen der lichtemittierenden Komponente (106) und dem lichtempfangenden Messwandler (51) in Abhängigkeit von der Verschiebung zwischen dem ersten und dem zweiten Befestigungspunkt zunehmend zu verdecken.

5. Sensoreinrichtung nach einem der Ansprüche 3 bis 4, wobei die Sensorvorrichtung (2; 102; 2002) ferner eine Lichtleitfaser (50) umfasst, die sich mindestens teilweise zwischen der lichtemittierenden Komponente (6) und dem lichtempfangenden Messwandler (140) erstreckt.

6. Sensoreinrichtung nach Anspruch 5, wobei der zweite Befestigungspunkt mit einem Punkt auf der Lichtleitfaser (50) gekoppelt ist, sodass die Bewegung des zweiten Befestigungspunkts relativ zum ersten Befestigungspunkt bewirkt, dass die Lichtleitfaser sich biegt, sodass gegenüberliegende Enden der Lichtleitfaser näher zueinander gezogen oder weiter auseinander gedrückt werden.

7. Sensoreinrichtung nach Anspruch 5, wobei die Sensorvorrichtung (102; 2002) eine erste Lichtleitfaser (45; 2051) und eine zweite Lichtleitfaser (44; 2030) umfasst, wobei die erste Lichtleitfaser dafür eingerichtet ist, von der lichtemittierenden Komponente (106; 2041) emittiertes Licht zu empfangen und das Licht zu der zweiten Lichtleitfaser zu übertragen, und wobei die Sensorvorrichtung so konfiguriert ist, dass eine erhöhte Verschiebung zwischen dem ersten und dem zweiten Befestigungspunkt eine relative seitliche Bewegung zwischen einem lichtemittierenden Ende der ersten Lichtleitfaser und einem lichtempfangenden Ende der zweiten Lichtleitfaser bewirkt, sodass weniger Licht von der ersten Lichtleitfaser zu der zweiten Lichtleitfaser übertragen wird.

8. Sensoreinrichtung nach einem der vorhergehenden Ansprüche, wobei die Sensorvorrichtung (302) ferner umfasst: eine mit dem ersten Punkt gekoppelte erste Kondensatorplatte (1250) und eine mit dem zweiten Punkt gekoppelte zweite Kondensatorplatte (1470) zum Bereitstellen einer variablen Kapazität zwischen der ersten und der zweiten Kondensatorplatte, wobei die Sensorvorrichtung dafür konfiguriert ist, die variable Kapazität zwischen der ersten und der zweiten Kondensatorplatte zu verwenden, um eine Verschiebung zwischen dem ersten und dem zweiten Befestigungspunkt zu erfassen.

9. Sensoreinrichtung nach einem der vorhergehenden Ansprüche, wobei die Sensorvorrichtung (302; 402) dafür konfiguriert ist, einen variablen Widerstand oder ein variables Magnetfeld oder ein variables elektrisches Feld zu verwenden, um eine Verschiebung zwischen dem ersten und dem zweiten Befestigungspunkt zu erfassen.

10. Sensoreinrichtung nach einem der vorhergehenden Ansprüche, ein Vorspannelement (65) umfassend, das dafür eingerichtet ist, einer Verschiebung zwischen dem ersten Befestigungspunkt und dem zweiten Befestigungspunkt Widerstand entgegenzusetzen.

11. Sensoreinrichtung nach einem der vorhergehenden Ansprüche, wobei die Steuerungseinrichtung dafür konfiguriert ist, einen geschätzten Druck zu berechnen, der durch das Kompressionskleidungsstück auf einen Träger des Kompressionskleidungsstücks ausgeübt wird, und zwar durch:

Bestimmen eines relativen oder absoluten Verschiebungswerts aus den Informationen, welche die erfasste Verschiebung darstellen;
Bestimmen eines relativen oder absoluten Spannkraftwerts aus den Informationen, welche die erfasste Verschiebung darstellen;
Berechnen des geschätzten Drucks durch Dividieren des bestimmten Spannkraftwerts durch eine lineare Funktion des bestimmten Verschiebungswerts.

12. Sensoreinrichtung nach Anspruch 11, wobei die lineare Funktion die Summe aus einer ersten Konstanten und einem Produkt ist, wobei das Produkt das Produkt aus einer zweiten Konstanten und dem bestimmten Verschiebungswert ist, und wobei die erste Konstante gleich dem Umfang des Kompressionskleidungsstücks in einem nicht gedehnten Zustand ist.

13. Kompressionskleidungsvorrichtung, umfassend ein Kompressionskleidungsstück (1; 720) und eine Sensoreinrichtung nach einem der vorhergehenden Ansprüche, wobei die Sensorvorrichtung (2; 102; 202; 302; 402; 710; 1602; 2002) der Sensoreinrichtung an einer Außenseite des Kompressionskleidungsstücks angebracht ist.

14. Kompressionskleidungsvorrichtung nach Anspruch 13, wobei das Kompressionskleidungsstück eine Vielzahl von Schichten umfasst und die Sensorvorrichtung an einer Außenseite einer Schicht des Kompressionskleidungsstücks angebracht ist.

15. Kompressionskleidungsvorrichtung nach einem der Ansprüche 13 bis 14, umfassend eine oder mehrere weitere Sensorvorrichtungen, wobei jede weitere Sensorvorrichtung umfasst:

einen jeweiligen ersten Befestigungspunkt zum Anbringen an einem jeweiligen ersten Punkt auf der Außenseite des Kompressionskleidungsstücks, und
einen jeweiligen zweiten Befestigungspunkt zum Anbringen an einem jeweiligen zweiten Punkt auf der Außenseite des Kompressionskleidungsstücks,
wobei jede weitere Sensoreinrichtung dafür eingerichtet ist, eine Verschiebung zwischen den jeweiligen ersten und zweiten Befestigungspunkten zu erfassen;
und wobei die Steuerungseinrichtung dafür konfiguriert ist, aus den jeweiligen erfassten Verschiebungen eine Blutflussrate oder eine Änderung des Muskelvolumens zu bestimmen.

**Revendications**

1. Appareil de capteur comprenant un dispositif de capteur (2 ; 102 ; 202 ; 302 ; 402 ; 710 ; 1602 ; 2002), destiné à être attaché à une surface externe d'un vêtement de compression (1 ; 720), et un dispositif de commande, dans lequel le dispositif de capteur comprend :

un premier point de montage destiné à être attaché à un premier point sur la surface externe du vêtement de compression ; et
un deuxième point de montage destiné à être attaché à un deuxième point sur la surface externe du vêtement de compression,
dans lequel le dispositif de capteur est agencé pour détecter un déplacement entre les premier et deuxième points de montage ; et
dans lequel le dispositif de commande est configuré pour traiter des informations représentatives du déplacement détecté pour estimer une pression exercée par le vêtement de compression sur une personne qui porte le vêtement de compression.

2. Appareil de capteur selon la revendication 1, dans lequel le dispositif de capteur (2 ; 102 ; 202 ; 302 ; 402 ; 710 ; 720 ; 1602 ; 2002) est agencé pour être attaché de façon amovible non destructrice au vêtement de compression.

3. Appareil de capteur selon l'une quelconque des revendications 1 ou 2, dans lequel le dispositif de capteur (2 ; 102 ; 202 ; 710 ; 1602 ; 2002) comprend en outre un composant émetteur de lumière (6 ; 106 ; 240 ; 2041) et un transducteur récepteur de lumière (140 ; 51 ; 345 ; 2022) et le dispositif de capteur est configuré de telle sorte qu'une augmentation du déplacement entre les premier et deuxième points de montage ait pour effet que moins de lumière arrive sur le transducteur récepteur de lumière, ce qui conduit à un signal de sortie plus faible en provenance du transducteur.

4. Appareil de capteur selon la revendication 3, dans lequel le dispositif de capteur (2 ; 102) comprend un élément interrupteur de lumière (31) configuré pour occlure progressivement un chemin optique entre le composant émetteur de lumière (106) et le transducteur récepteur de lumière (51) en fonction du déplacement entre les premier et deuxième points de montage.

5. Appareil de capteur selon l'une quelconque des revendications 3 à 4, dans lequel le dispositif de capteur (2 ; 102 ; 2002) comprend en outre une fibre optique (50) étendue au moins partiellement entre le composant émetteur de lumière (6) et le transducteur récepteur de lumière (140).

**6.** Appareil de capteur selon la revendication 5, dans lequel le deuxième point de montage est couplé à un point sur la fibre optique (50) de telle sorte que le mouvement du deuxième point de montage par rapport au premier point de montage provoque un fléchissement de la fibre optique de telle sorte que des extrémités opposées de la fibre optique soient tirées l'une vers l'autre ou poussées à l'écart l'une de l'autre.

**7.** Appareil de capteur selon la revendication 5, dans lequel le dispositif de capteur (102 ; 2002) comprend une première fibre optique (45 ; 2051) et une deuxième fibre optique (44 ; 2030), dans lequel la première fibre optique est agencée pour recevoir de la lumière émise depuis le composant émetteur de lumière (106 ; 2041) et pour transmettre la lumière à la deuxième fibre optique, et dans lequel le dispositif de capteur est configuré de telle sorte qu'une augmentation du déplacement entre les premier et deuxième points de montage génère un mouvement latéral relatif entre une extrémité émettrice de lumière de la première fibre optique et une extrémité réceptrice de lumière de la deuxième fibre optique de telle sorte que moins de lumière soit transmise de la première fibre optique à la deuxième fibre optique.

**8.** Appareil de capteur selon l'une quelconque des revendications précédentes, dans lequel le dispositif de capteur (302) comprend en outre une première plaque de condensateur (1250) couplée au premier point et une deuxième plaque de condensateur (1470) couplée au deuxième point, pour fournir une capacité variable entre les première et deuxième plaques de condensateur, dans lequel le dispositif de capteur est configuré pour utiliser la capacité variable entre les première et deuxième plaques de condensateur pour détecter un déplacement entre les premier et deuxième points de montage.

**9.** Appareil de capteur selon l'une quelconque des revendications précédentes, dans lequel le dispositif de capteur (302 ; 402) est configuré pour utiliser une résistance variable ou un champ magnétique variable ou un champ électrique variable pour détecter un déplacement entre les premier et deuxième points de montage.

**10.** Appareil de capteur selon l'une quelconque des revendications précédentes, comprenant un élément de contrainte (65) agencé pour résister à un déplacement entre le premier point de montage et le deuxième point de montage.

**11.** Appareil de capteur selon l'une quelconque des revendications précédentes, dans lequel le dispositif de commande est configuré pour calculer une pression estimée exercée par le vêtement de compression sur une personne qui porte le vêtement de compression en :

déterminant une valeur de déplacement relatif ou absolu à partir des informations représentatives du déplacement détecté ;
déterminant une valeur de force de tension relative ou absolue à partir des informations représentatives du déplacement détecté ; et
calculant la pression estimée en divisant la valeur de force de tension déterminée par une fonction linéaire de la valeur de déplacement déterminée.

**12.** Appareil de capteur selon la revendication 11, dans lequel la fonction linéaire est la somme d'une première constante et d'un produit, dans lequel le produit est le produit d'une deuxième constante et de la valeur de déplacement déterminée et dans lequel la première constante est égale à la circonférence du vêtement de compression dans un état non tendu.

**13.** Appareil à vêtement de compression comprenant un vêtement de compression (1 ; 720) et un appareil de capteur tel que revendiqué selon l'une quelconque des revendications précédentes, dans lequel le dispositif de capteur (2 ; 102 ; 202 ; 302 ; 402 ; 710 ; 1602 ; 2002) de l'appareil de capteur est attaché à une surface externe du vêtement de compression.

**14.** Appareil à vêtement de compression selon la revendication 13, dans lequel le vêtement de compression comprend une pluralité de couches et le dispositif de capteur est attaché à une surface externe d'une couche du vêtement de compression.

**15.** Appareil à vêtement de compression selon l'une quelconque des revendications 13 à 14, comprenant un ou plusieurs dispositifs de capteur supplémentaires, chaque dispositif de capteur supplémentaire comprenant :

un premier point de montage respectif destiné à être attaché à un premier point respectif sur la surface externe du vêtement de compression ; et
un deuxième point de montage respectif destiné à être attaché à un deuxième point respectif sur la surface externe du vêtement de compression ;

dans lequel chaque dispositif de capteur supplémentaire est agencé pour détecter un déplacement entre les premier et deuxième points de montage respectifs ;

et dans lequel le dispositif de commande est configuré pour déterminer un débit d'écoulement sanguin ou une variation de volume musculaire à partir des déplacements détectés respectifs.

FIG. 1

FIG. 2a

FIG. 2b

FIG. 2c

FIG. 2d

FIG. 3

FIG. 4a

FIG. 4b

FIG. 4c

FIG. 4d

240

235

345

460

$X_1$

202

FIG. 5a

240

235

345

460

$X_2$

202

FIG. 5b

FIG. 6a

FIG. 6b

*1155*　　　*1121*　　　*1119*　　　*1130*

*402*

*1120*　　　FIG. 7

*1130*　　　*1140*

*840*

*800*

*820*

*815*

*806*

*1120*

FIG. 8

FIG. 9

FIG. 10

FIG. 11

FIG. 12a

FIG. 12b

FIG. 12c

FIG. 12d

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20170079868 A1
- US 20190151160 A1
- US 20170100300 A1